# EUROPEAN PATENT SPECIFICATION

(11) **EP 4 616 416 B1**
(45) Date of publication and mention of the grant of the patent: **19.08.2026**
(21) Application number: 22822760.9
(22) Date of filing: 09.11.2022
(51) Int. Cl.: G16H 20/17, A61M 5/142, A61M 5/168, A61M 5/36

(54) **AIR-IN-LINE PREVENTION ALGORITHM**
LUFT-IN-LINE-VORBEUGUNGSALGORITHMUS
ALGORITHME DE PRÉVENTION DE PRÉSENCE D'AIR DANS UNE CONDUITE

(43) Date of publication of application: 17.09.2025
(73) Proprietor: CareFusion 303, Inc., San Diego, CA 92130 (US)
(72) Inventor: WORKMAN, Michael, K., Carlsbad, CA 92010 (US); HORVATH, Susan, Fort Worth, TX 76244 (US)
(74) Representative: Zenz Patentanwälte Partnerschaft mbB
(86) International application number: PCT/US2022/049455
(87) International publication number: WO 2024/102124

(56) References cited:
- EP-A1- 3 003 429
- US-A1- 2013 226 130
- US-A1- 2014 350 513
- US-A1- 2014 358 077

## Description

### BACKGROUND

Fluid infusion pumps operatively engage with fluid administration sets to deliver medication or drugs to patients from fluid supplies according to infusion programs. In some scenarios, the infusion program specifies a volume to be infused (VTBI) that is greater than a container volume of fluid present in the fluid supply. On these occasions, towards the end of the infusion, volume no longer remains and, because the pump continues to operate, air may be sucked into the tubing of the fluid administration set. Air in the tubing can be harmful if it is pumped into the patient.

EP 3 003 429 Al discloses an infusion system for being operatively connected to a fluid delivery line and to an infusion container including a pump, a plurality of different types of sensors connected to the pump or the fluid delivery line, at least one processor, and a memory. The plurality of different types of sensors are configured to indicate whether air is in the fluid delivery line. The memory includes programming code for execution by the at least one processor. The programming code is configured to, based on measurements taken by the plurality of different types of sensors, determine: whether there is air in the fluid delivery line; whether there is a partial occlusion or a total occlusion in the fluid delivery line; or a percentage of the air present in the fluid delivery line or the probability of the air being in the fluid delivery line.

US 2014/350513 A1 discloses An infusion system for being operatively connected to a fluid delivery line and to an infusion container includes a pump, a plurality of different types of sensors connected to the pump or the fluid delivery line, at least one processor, and a memory. The plurality of different types of sensors are configured to indicate whether air is in the fluid delivery line. The memory includes programming code for execution by the at least one processor. The programming code is configured to, based on measurements taken by the plurality of different types of sensors, determine: whether there is air in the fluid delivery line; whether there is a partial occlusion or a total occlusion in the fluid delivery line; or a percentage of the air present in the fluid delivery line or the probability of the air being in the fluid delivery line.

US 2014/358077 Al discloses a method for infusing an infusion fluid. In one step of a method for infusing an infusion fluid, the infusion fluid is pumped through a fluid delivery line of an infusion system. In another step, measurements are taken with at least one sensor connected to the infusion system. In an additional step, an air determination is determined with at least one processor. The air determination is related to air in the fluid delivery line. The air determination is based on the measurements taken by the at least one sensor. The air determination is further based on: (1) medication information regarding the infusion fluid or infusion information regarding the infusion of the infusion fluid; or (2) multi-channel filtering of the measurements from the at least one sensor or non-linear mapping of the measurements from the at least one sensor; and statistical process control charts applied to the multi-channel filtered measurements or applied to the non-linear mapped measurements.

US 2013/226130 A1 discloses systems and methods for sensing bubbles during fluid infusion. An infusion device comprises a pathway, a pump adjacent the pathway, one or more bubble sensors, and first and second modules. The pump alters the pathway to pump fluid through tubing received in the pathway. The bubble sensors are configured to detect bubbles in the fluid being pumped through the tubing. The first module is configured to generate a first alarm condition when the bubble sensors detect a bubble having a first volume above a first preselected threshold. The second module is configured to generate a second alarm condition when the bubble sensors detect a plurality of bubbles over a preselected period of time having a combined volume above a second preselected threshold. A third module may be also be used to record a total combined volume of bubbles detected by the bubble sensors during an infusion event.

To avoid this, clinicians may short program the VTBI on the infusion programs so the pump stops early to avoid air from entering the system. Short programming results in clinicians programming less VTBI than what is ordered. When the infusion stops, the clinician returns to the pump, estimates the remaining bag volume, reprograms the VTBI to be less than the estimate, restarts the infusion, and goes to retrieve another bag. This solution can be wasteful (e.g., pump resources are spent to alarm under nominal conditions and/or to program and reprogram the pump), can interrupt infusions, and can lead to under dosing the total dose to be administered.

### SUMMARY

Based on the above discussion, there is a need for an infusion pump capable of preventing or prolonging the accidental introduction of air in the system without requiring the clinician to change the VTBI of the infusion program. The infusion pumping systems and methods described herein dynamically escalate notifications, dynamically decrease pump rates, and dynamically increase air-in-line (AIL) detection sensitivity levels based on the amount of pumped or remaining VTBI for a given infusion program. Such systems and methods balance the various trade-offs involving nuisance alarms, pumping efficiency, and patient safety.

The invention is defined in the independent claims. Preferable embodiments are further laid out in the dependent claims. In one aspect, some implementations include a infusion device, comprising: a pumping element configured to pump a fluid through a fluid tube into a patient connector; an air-in-line sensor configured to measure air within the fluid tube; a display; a processor; and a non-transitory computer readable medium comprising instructions that, when executed by the processor, cause the infusion device to: receive an instruction indicating a total volume of a fluid to pump; cause the pumping element to pump the fluid through the fluid tube according to a pumping rate; cause the air-in-line sensor to measure an amount of air within the fluid tube according to a sensitivity level; determine that a first amount of the total volume of the fluid has been pumped or that a second amount of the total volume of the fluid remains to be pumped; in accordance with the determination that the first amount has been pumped or that the second amount remains to be pumped, output a notification on the display and/or adjust the pumping element to reduce the pumping rate; determine that a third amount of the total volume of the fluid has been pumped or that a fourth amount of the total volume of the fluid remains to be pumped, wherein the third amount is higher than the first amount and the fourth amount is lower than the second amount; and in accordance with the determination that the third amount has been pumped or that the fourth amount remains to be pumped, increase the sensitivity level of the air-in-line sensor.

In another aspect, some implementations include a method of operating an infusion device to reduce air-in-line alarms, the method comprising: receiving an instruction at a processor of the infusion device indicating a total volume of a fluid for a pumping element of the infusion device to pump through a fluid tube into a patient connector; causing the pumping element to pump the fluid through the fluid tube according to a pumping rate; causing an air-in-line sensor of the infusion device to measure an amount of air within the fluid tube according to a sensitivity level; determining that a first amount of the total volume of the fluid has been pumped or that a second amount of the total volume of the fluid remains to be pumped; in accordance with the determination that the first amount has been pumped or that the second amount remains to be pumped, outputting a notification on the display and/or adjusting the pumping element to reduce the pumping rate; determining that a third amount of the total volume of the fluid has been pumped or that a fourth amount of the total volume of the fluid remains to be pumped, wherein the third amount is higher than the first amount and the fourth amount is lower than the second amount; and in accordance with the determination that the third amount has been pumped or that the fourth amount remains to be pumped, increasing the sensitivity level of the air-in-line sensor.

### BRIEF DESCRIPTION OF THE DRAWINGS

For a better understanding of the various described implementations, reference should be made to the Detailed Description below, in conjunction with the following drawings. Like reference numerals refer to corresponding parts throughout the drawings.
Figure 1 depicts an example patient care system that includes an infusion device in accordance with some implementations.
Figure 2 depicts an internal view of an infusion pump of the infusion device of Figure 1, including an air-in-line (AIL) detector in accordance with some implementations.
Figure 3 depicts a table of AIL preventative actions and corresponding thresholds in accordance with some implementations.
Figure 4 depicts a graph of AIL preventative actions and corresponding thresholds in accordance with some implementations.
Figure 5 depicts an example AIL prevention process in accordance with some implementations.
Figure 6 depicts an example AIL prevention process including dynamic adjustment of notification escalation, pump rate, and AIL detection sensitivity in accordance with some implementations.
Figure 7 depicts an example AIL prevention process including dynamic adjustment of pump rate and AIL detection sensitivity in accordance with some implementations.
Figure 8 depicts an example AIL prevention process including dynamic adjustment of pump rate in accordance with some implementations.
Figure 9 depicts an example AIL prevention process including dynamic adjustment of AIL detection sensitivity in accordance with some implementations.
Figure 10 is a conceptual diagram illustrating an example electronic system in accordance with some implementations.

### DETAILED DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In some instances, well-known methods, procedures, components, circuits, and networks have not been described in detail so as not to unnecessarily obscure aspects of the implementations.

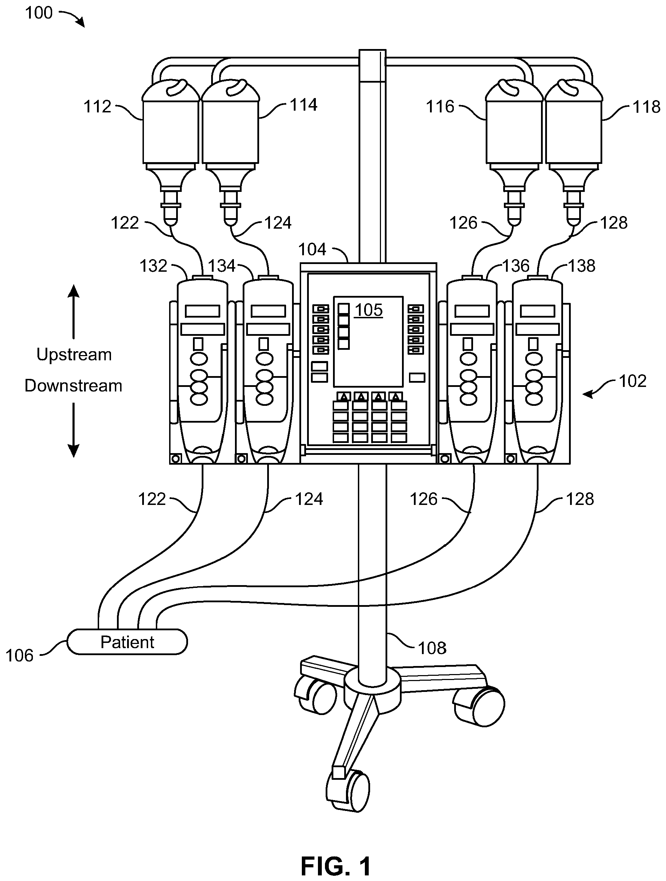

Figure 1 depicts an example patient care system 100 that includes an infusion device 102, in accordance with some implementations. The patient care system 100 includes four fluid infusion pumps 132, 134, 136, and 138, each of which is in operative engagement with a respective fluid administration set 122, 124, 126, and 128. In the depicted example, the infusion pumps are connected to and controlled by a mainframe infusion controller 104. Fluid supplies 112, 114, 116, and 118, which may take various forms but in this case are shown as bottles, are inverted and suspended above the pumps. Fluid supplies 112, 114, 116, and 118 may also take the form of bags or other types of containers. Both the infusion device 102 and the fluid supplies 112, 114, 116, and 118 are mounted to a roller stand or pole 108. The specific fluid supplies 112, 114, 116, and 118, as well as their orientation (e.g., mount location, mount height, mounting type, etc.) within the care area, may generate one or more interaction records. The interaction record for a set, for example, may be generated in part by detecting a scannable code associated with the set or detecting a physical structure on the set that encodes identifying information for the set prior to use.

As shown in the example implementation, each administration set 122, 124, 126, and 128 connects a respective fluid supply 112, 114, 116, and 118 to a patient 106 so that the patient 106 may receive the fluids in all the fluid supplies 112, 114, 116, and 118. The administration set may be identified either actively by, for example, scanning by a clinician or passively by, for example, wireless or optical detection of the administration set.

In the depicted example, a separate infusion pump 132, 134, 136, and 138 is used to infuse each of the fluids of the fluid supplies 112, 114, 116, and 118 into the patient 106. The infusion pumps 132, 134, 136, and 138 are flow control devices that will act on the respective tube or fluid conduit of the fluid administration set to move the fluid from the fluid supply through the conduit and to the patient 106. Because individual infusion pumps 132, 134, 136, and 138 are used, each pump may be individually set to the pumping or operating parameters required for infusing the particular medical fluid from the respective fluid supply into the patient at the particular rate prescribed for that fluid by the clinician.

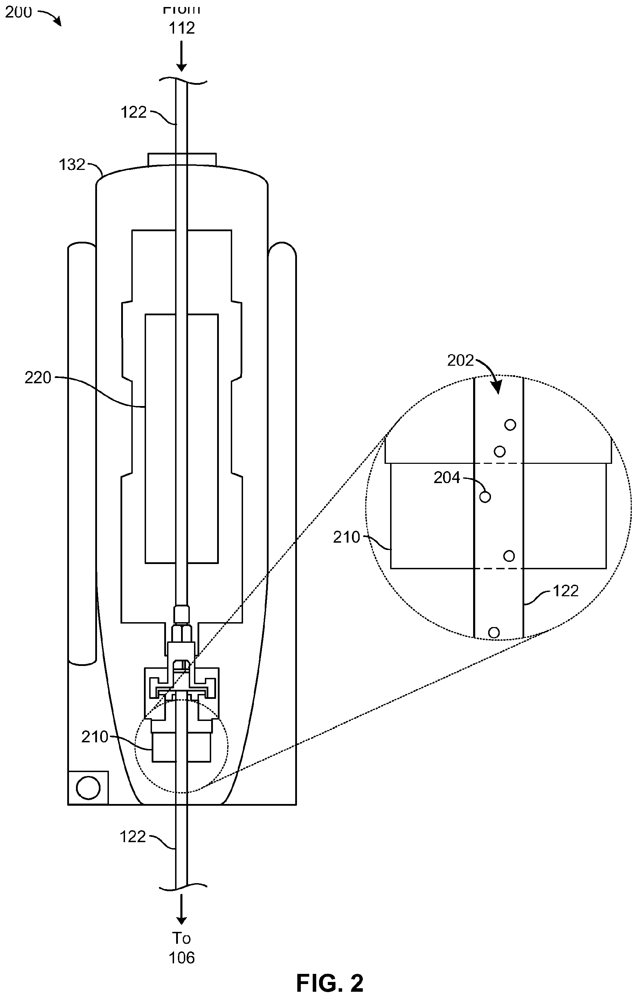

In some implementations, each fluid infusion pump 132, 134, 136, and 138 includes an air-in-line (AIL) detector (also referred to as an air detector, an air sensor, or an AIL sensor), described in more detail below with reference to Figure 2 (sensor 210). The AIL detectors are configured to detect air in the tubing of the administration sets 122, 124, 126, and 128. The AIL detectors may be located inside respective fluid infusion pumps 132, 134, 136, and 138 (e.g., as shown in Figure 2), or in other implementations, upstream or downstream of the fluid infusion pumps 132, 134, 136, and 138, as long as a portion of each administration set 122, 124, 126, and 128 passes through a respective AIL detector.

The AIL detectors analyze one or more AIL metrics of the respective fluids as they flow through the respective administration sets 122, 124, 126, and 128. For example, the AIL metric may measure a quantity of air, such as a number of air bubbles. The AIL metric may be measured at intervals or for a time series by sampling values over time. When more than one AIL values are collected for analysis, the AIL metric may reflect an average, a moving average, a maximum for period, a minimum for period, correspondence to a threshold or range, or the like, of the collected AIL values.

The infusion device 102 may be configured to adjust its operation in accordance with the AIL metrics. For example, if an AIL metric exceeds a safety threshold (e.g., a maximum number of air bubbles within a given amount of time or volume of liquid), the infusion device 102 may pause a pumping operation of the infusion pump (e.g., 132) corresponding to the AIL detector that measured the AIL metric exceeding the safety threshold. The infusion device 102 may also output a notification to notify a clinician that the safety threshold has been exceeded. Specifically, the controller 104 or a specific infusion pump 132, 134, 136, or 138 may output the notification on a display (e.g., display 105 on the controller 104 or a display on a specific infusion pump), output the notification as an audio alert from a speaker, and/or transmit the notification to another computing device communicatively coupled to the controller 104 (e.g., external equipment such as a medical facility server or other computer and with a portable processor, such as a handheld communication device or a laptop-type of computer, or other information device that a clinician may have access to).

Typically, medical fluid administration sets have more parts than are shown in Figure 1. Many have check valves, drip chambers, valved ports, connectors, and other devices well known to those skilled in the art. These other devices have not been included in the drawings so as to preserve clarity of illustration.

Each infusion pump (e.g., 132) includes a door and a handle that operates to lock the door in a closed position for operation and to unlock and open the door for access to the internal pumping and sensing mechanisms and to load administration sets for the pump. When the door is open, the tube of a respective administration set (e.g., 122) can be connected with the pump (e.g., 132). When the door is closed, the tube is brought into operating engagement with the pumping mechanism, upstream and downstream pressure sensors, and other equipment of the pump. A display, such as an LED display, is located in plain view on the door in this implementation and may be used to visually communicate various information relevant to the pump 136, such as alert indications (e.g., alarm messages).

Control keys exist on each infusion pump for programming and controlling operations of the infusion pump as desired. In some implementations, the control keys may be presented as interactive elements on the display (e.g., a touchscreen display). The infusion device 102 and/or the infusion pumps may also include audio alert equipment in the form of a speaker (not shown).

The infusion controller 104 of the infusion device 102 includes a display 105 for visually communicating various information, such as the operating parameters of a connected pump and alert indications and alert messages, and control keys for selecting and/or setting control parameters and/or options for controlling the infusion device 102 and connected modules. The infusion controller 104 may also include a speaker to provide audible alerts. In some implementations, the display 105 may be implemented as a touchscreen display. In such implementations, the control keys may be omitted or reduced in number by providing corresponding interactive elements via a graphical user interface presented via the display 105. In some implementations, each control key may select a corresponding option displayed in the display 105.

The infusion controller 104 may include a communications system (not shown) with which the infusion controller 104 may communicate with external equipment such as a medical facility server or other computer and with a portable processor, such as a handheld communication device or a laptop-type of computer, or other information device that a clinician may have to transfer information as well as to download drug libraries (e.g., to infusion pump 132). The communication module may be used to transfer access and interaction information for clinicians encountering the infusion controller or device coupled therewith (e.g., pump 132, a bar code scanner). The communications system may include one or more of a radio frequency (RF) system, an optical system (e.g., infrared), a Bluetooth system, or other wired or wireless system. The bar code scanner and communications system may alternatively be included integrally with the infusion pump 132, such as in cases where an infusion controller 104 is not used, or in addition to one with the infusion controller 104. Further, information input devices need not be hard-wired to medical instruments; information may be transferred through a wireless connection as well.

Additionally, other types of modules may be connected to the pump modules or to the infusion controller 104, such as a syringe pump module, a patient controlled analgesic module, an external safety device (ESD), a patient safety device such as an end tidal CO₂ monitoring module or an oximeter monitoring module, or the like. Some ESDs may include, for example, an air trap, an air detector (e.g., an AIL detector as described herein), a flow detector, a particulate detector, or a spectral analyzer.

In some implementations, AIL measurements from AIL detectors are transmitted to a server or other coordination device, and the methods disclosed herein are implemented on the server or other coordination device. For example, more sophisticated and computationally intensive approaches like machine-learning can be implemented on the server (or on a PCU with a larger memory and/or CPU resources). In some implementations, machine learning is used to identify AIL conditions based on AIL measurements received from the pumps.

Figure 2 depicts an internal view of an infusion pump 132 of the infusion device 102 of Figure 1, including an AIL detector 210 in accordance with some implementations. Tubing of the administration set 122 (also referred to as hose, line, or conduit) is fed through infusion pump 132 by placing a portion of the tubing proximate to a pumping element 220, which is configured to pump fluid from the fluid supply 112 to a patient 106 by way of a patient connector (e.g., at an injection site coupled to the patient).

Pumping element 220 pumps fluid from the fluid supply 112 through an AIL detector 210 before the fluid reaches the patient 106. As a result, air detected in the tubing of the administration set 122 may be prevented from reaching the patient 106 upon, for example, the AIL detector 210 sending a notification to processing circuitry in the pump 132 indicating an amount of detected air having exceeded a safety threshold, and the processing circuitry pausing the pumping element 220.

The pumping element 220 comprises any pumping structure, such as a linear peristaltic pump, configured to cause fluid to be directed from one end of a tube to another end of the tube at an adjustable pump rate. The pump 132 may dynamically adjust the pump rate of the pumping element 220 based on an amount of fluid that has been pumped or remains to be pumped for a given VTBI. As the amount of pumped fluid approaches the specified VTBI, the pump 132 may reduce the pump rate.

By reducing the pump rate in such a manner, accidental introduction of air into the administration set 122 (e.g., caused by the VTBI being greater than the volume of the fluid supply 112) is prolonged and accompanying risks are abated. As the amount of fluid pumped continues to approach the end of the VTBI, the pump rate may continue to be decreased, and in some implementations, the pumping may eventually be paused. By dynamically adjusting the pump rate in such a manner, the pump 132 may optimize the trade-off between treatment time (using a relatively high pump rate towards the beginning of the VTBI) and AIL prevention (using a relatively low pump rate towards the end of the VTBI).

The AIL detector 210 comprises an air sensor, such as an optical or ultrasonic air bubble detector, configured to detect air (e.g., air bubbles 204) in a liquid (e.g., fluid 202) at an adjustable detection sensitivity. In some implementations, the AIL detector 210 may count a rolling average number of air bubbles and output an alarm if any average number of detected air bubbles within a given sample period exceeds a safety threshold. In some implementations, the AIL detector 210 may count an accumulated number of air bubbles from the beginning of the infusion program and output an alarm if a total number of detected air bubbles exceeds a safety threshold. In some implementations, the AIL detector 210 may count air bubbles or detect a volume of air using any other suitable method and output an alarm if the detected number of air bubbles or the detected volume of air exceeds a safety threshold.

The pump 132 may dynamically adjust the detection sensitivity of the AIL detector 210 based on an amount of fluid that has been pumped or remains to be pumped for a given VTBI. As the amount of pumped fluid approaches the specified VTBI, the pump 132 may increase the detection sensitivity of the AIL detector 210. In some implementations, the pump 132 may increase the detection sensitivity of the AIL detector 210 by lowering the safety threshold and/or by shortening the sample period (and thus decreasing the sample size).

By increasing the detection sensitivity of the AIL detector 210 in such a manner, accidental introduction of air into the administration set 122 (e.g., caused by the VTBI being greater than the volume of the fluid supply 112) is prolonged and accompanying risks are abated. By dynamically adjusting the detection sensitivity in such a manner, the pump 132 may optimize the trade-off between prevention of nuisance alarms caused by false positive AIL detections (using a relatively low detection sensitivity towards the beginning of the VTBI) and AIL prevention (using a relatively high detection sensitivity towards the end of the VTBI).

Stated another way, when the infusion program commences, nuisance alarm prevention may be initially be prioritized by keeping the AIL detection sensitivity relatively low, or otherwise at a reasonable rate that optimizes for air detection while minimizing false positives. However, as the infusion program nears the end of the infusion program, nuisance alarm prevention is not as important as the increasing risk of air being introduced into the administrative set 122 (e.g., due to the fluid supply 112 running out before the VTBI has been reached). Thus, toward the end of the infusion program, AIL detection is prioritized by increasing detection sensitivity, even if that means an increase in false positive AIL alarms. These false positive alarms may even be useful in that they could serve as the basis for additional reminders to the clinician when preventative measures should be taken to avoid accidental air in the system (e.g., by switching out the fluid supply 112 before it runs out).

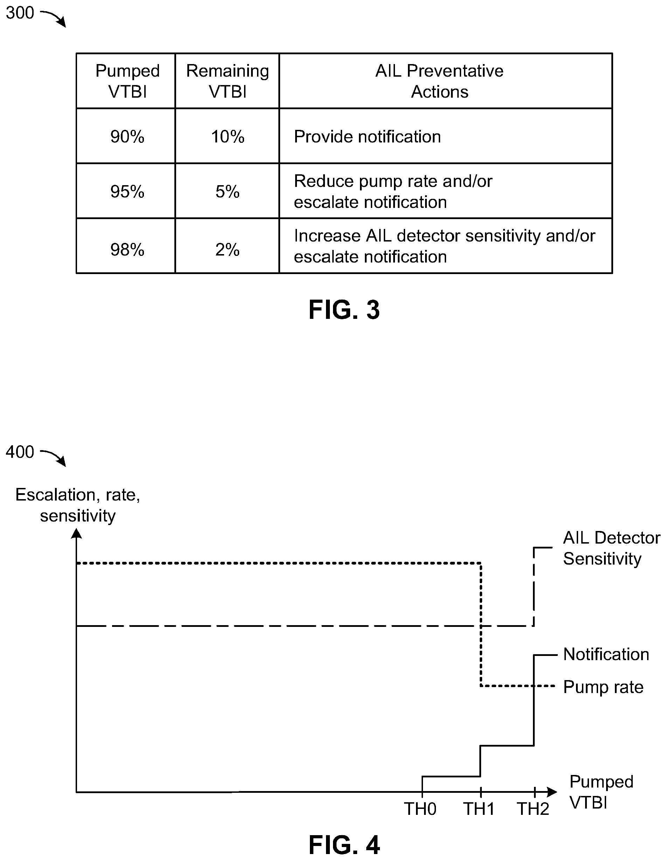

Figure 3 depicts a table 300 of AIL preventative actions and corresponding thresholds in accordance with some implementations. In the example depicted in table 300, when an initial threshold (e.g., 90%) of the VTBI has been pumped (or, likewise, when 10% of the VTBI remains to be pumped), pump 132 outputs a notification indicating the VTBI is within 10% of being reached, and that preventative measures should be taken to avoid accidental air in the system.

When a first threshold after the initial threshold (e.g., 95%) of the VTBI has been pumped (or, likewise, when 5% of the VTBI remains to be pumped), pump 132 reduces the pump rate of pumping element 220, as described above, to prolong the accidental introduction of air in the system. At this time, pump 132 may additionally or alternatively escalate the notification (e.g., by emphasizing a visual alarm on the display, by increasing the volume of an audible alarm, and/or by transmitting the notification to additional computing devices).

When a second threshold after the initial threshold (e.g., 98%) of the VTBI has been pumped (or, likewise, when 2% of the VTBI remains to be pumped), pump 132 increases the detection sensitivity of the AIL detector 210, as described above, to increase the likelihood of a timely alarm in the event air is detected in the administration set 122, thereby preventing the accidental introduction of air in the system. At this time, pump 132 may additionally or alternatively further escalate the notification (e.g., by further emphasizing a visual alarm on the display, by further increasing the volume of an audible alarm, and/or by further transmitting the notification to additional computing devices).

The threshold values and corresponding actions in table 300 are provided for illustrative purposes, and they may differ depending on the implementation. The threshold values and corresponding actions may be configurable based on the specific infusion program, infusion type (e.g., continuous or fluid), and/or drug or medication.

For example, in some implementations, the initial threshold may be an amount between 75% and 95% of the VTBI (or less than 75% or greater than 95% in other implementations), corresponding to between 5% and 25% of the VTBI remaining to be pumped.

In some implementations, the first threshold after the initial threshold may be an amount between 90% and 97% of the VTBI (or less than 90% or greater than 97% in other implementations), corresponding to between 3% and 10% of the VTBI remaining to be pumped.

In some implementations, the second threshold after the initial threshold may be an amount between 95% and 99% of the VTBI (or less than 95% or greater than 99% in other implementations), corresponding to between 1% and 5% of the VTBI remaining to be pumped.

Figure 4 depicts a graph 400 of AIL preventative actions and corresponding thresholds in accordance with some implementations. The threshold values and corresponding actions in the graph are provided for illustrative purposes, and they may differ depending on the implementation. The threshold values and corresponding actions may be configurable based on the specific infusion program, infusion type (e.g., continuous or fluid), and/or drug or medication.

Before an initial threshold TH0 (e.g., 90%) of the VTBI has been reached, the pumping element 220 operates at an initial pump rate, the AIL detector 210 operates at an initial detection sensitivity, and no AIL-related notifications are provided. At the initial threshold TH0, a notification is provided at a first level of escalation while the pump rate and the AIL detection sensitivity remain unchanged. When a first threshold TH1 after the initial threshold (e.g., 95%) of the VTBI is reached, the notification escalation level is raised and the pump rate is decreased while the AIL detection sensitivity remains the same. When a second threshold TH2 after the initial threshold (e.g., 98%) of the VTBI is reached, the notification escalation level is raised, the pump rate remains the same, and the AIL detection sensitivity is increased.

The threshold values and corresponding actions in graph 400 are provided for illustrative purposes, and they may differ depending on the implementation. The threshold values and corresponding actions may be configurable based on the specific infusion program, infusion type (e.g., continuous or fluid), and/or drug or medication.

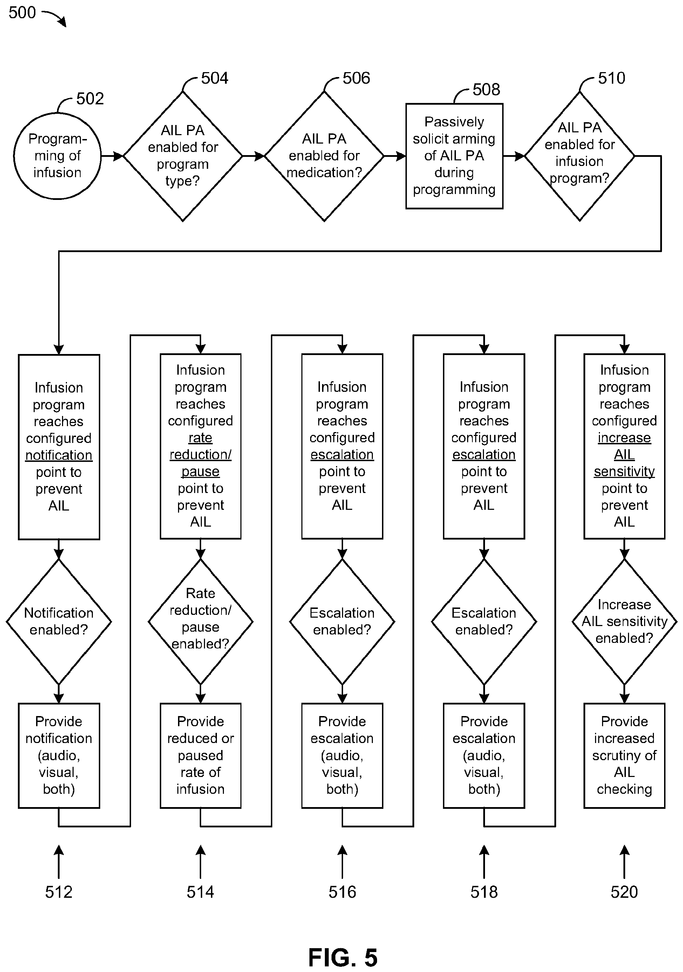

Figures 5-9 depict example AIL prevention processes 500-900 in accordance with some implementations. One or more blocks of processes 500-900 may be implemented, for example, by one or more computing devices, such as infusion device 102. In some implementations, one or more of the blocks may be implemented based on one or more machine learning algorithms. In some implementations, one or more of the blocks may be implemented apart from other blocks, and by one or more different processors or devices. Further, for explanatory purposes, the blocks of example processes 500-900 are described as occurring in serial, or linearly. However, multiple blocks of example processes 500-900 may occur in parallel. Additionally, the blocks of example processes 500-900 need not be performed in the order shown and one or more of the blocks of example processes 500-900 need not be performed.

Figure 5 depicts an example AIL prevention process 500 in accordance with some implementations. An infusion program (also referred to as an instruction) is received (502) at an infusion device (e.g., controller 104 or a pump 132). The infusion program specifies characteristics (e.g., indicating VTBI, program type, medication, pump rate, and/or pump timing) for an infusion program involving one or more of the infusion pumps 132, 134, 136, and 138. AIL prevention algorithms (AIL PA) may be enabled based on the program type (504) and the specified medication (506). In addition, the clinician may be prompted (e.g., passively solicited at an interface of the controller 104) to enable AIL PA (508) during the programming. If AIL PA is enabled by the clinician (510), process 500 continues with operations 512-520.

In response to the infusion program being received and the AIL PA being enabled, the infusion device begins the infusion program by causing a pumping element of the infusion device (e.g., 220) to pump the fluid through the fluid tube according to a pumping rate and subject to a safety threshold amount of air within the fluid tube; and by causing an AIL sensor of the infusion device (e.g., 210) to measure an amount of the air within the fluid tube according to a AIL sensitivity detection level (also referred to as a sensitivity level).

In operations 512, when the infusion program reaches a configured notification point (a first VTBI-related threshold) for preventing AIL, the controller 104 determines if notification functionality is enabled (e.g., based on the infusion program and specifications obtained from the clinician during programming), and if so, provides a notification (e.g., an audio notification, a visual notification, or both) as described above.

In operations 514, when the infusion program reaches a configured pump rate reduction or pause point (a second VTBI-related threshold) for preventing AIL, the controller 104 determines if pump rate reduction or pause functionality are enabled, and if so, reduces or pauses the pumping rate as described above. Enabling of pump rate reduction or pause may be based on one or more of: the infusion program, specifications obtained from the clinician during programming, the pump's location (e.g., clinical care area), drug being infused, or other value available to the infusion device.

In operations 516, when the infusion program reaches a configured notification escalation point (a third VTBI-related threshold) for preventing AIL, the controller 104 determines if notification escalations are enabled, and if so, provides an escalated notification (e.g., an audio notification, a visual notification, or both) as described above. Enabling of escalation may be based on one or more of: the infusion program, specifications obtained from the clinician during programming, the pump's location (e.g., clinical care area), drug being infused, or other value available to the infusion device.

In operations 518, when the infusion program reaches a configured notification escalation point (a fourth VTBI-related threshold) for preventing AIL, the controller 104 determines if notification escalations are enabled, and if so, provides an escalated notification (e.g., an audio notification, a visual notification, or both) as described above. Enabling of escalation may be based on one or more of: the infusion program, specifications obtained from the clinician during programming, the pump's location (e.g., clinical care area), drug being infused, or other value available to the infusion device.

In operations 520, when the infusion program reaches a configured AIL detection sensitivity increase point (a fifth VTBI-related threshold) for preventing AIL, the controller 104 determines if AIL detection sensitivity increases are enabled, and if so, provides increases the AIL detection sensitivity level of the AIL detector, thereby providing increased scrutiny of AIL checking. AIL detection sensitivity increases may be enabled based on one or more of: the infusion program, specifications obtained from the clinician during programming, the pump's location (e.g., clinical care area), drug being infused, or other value available to the infusion device.

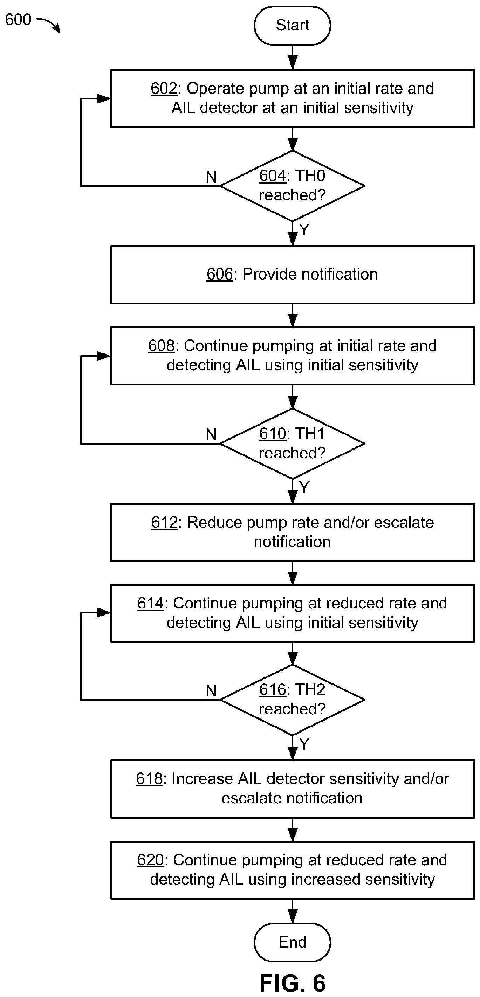

Figure 6 depicts an example AIL prevention process 600 including dynamic adjustment of notification escalation, pump rate, and AIL detection sensitivity in accordance with some implementations. The method starts upon receiving an infusion program (or an instruction associated with an infusion program) specifying a VTBI. At an initial step in the infusion program (602), the pumping element (e.g., 220) operates (pumps fluid through the fluid tube of the infusion device) at or according to a pumping rate (optionally referred to as an initial or first pumping rate), and the AIL detector (e.g., 210) (also referred to as an AIL sensor) operates (measures an amount of air within the fluid tube) at or according to a sensitivity level (optionally referred to as an initial or first sensitivity level).

While the infusion program runs with the aforementioned parameters, the pump determines (604) if an initial VTBI threshold TH0 is met (or reached or exceeded) (e.g., by sensing an amount of fluid that has been pumped or by determining how full or empty the fluid supply is). The initial VTBI threshold TH0 corresponds to a first predetermined amount of the VTBI having been pumped (e.g., 90%) or remaining to be pumped (e.g., 10%). When the initial VTBI threshold TH0 is met, the pump (e.g., 132) provides (606) a notification (audio, visual, or both) indicating so, and continues (608) pumping at the initial pump rate and detecting AIL using the initial AIL detection sensitivity level.

While the infusion program runs with the initial pump rate and the initial AIL detection sensitivity level, the pump determines (610) if a first VTBI threshold TH1 after the initial VTBI threshold is reached (e.g., by sensing an amount of fluid that has been pumped or by determining how full or empty the fluid supply is). The first VTBI threshold TH1 corresponds to a second predetermined amount of the VTBI having been pumped (e.g., 95%) higher than the first predetermined amount of the VTBI having been pumped, or a second predetermined amount of the VTBI remaining to be pumped (e.g., 5%) lower than the first predetermined amount of the VTBI remaining to be pumped. When the first VTBI threshold TH1 is reached, the pump (e.g., 132) reduces (612) the pump rate and/or escalates the notification, and continues (614) pumping at the reduced pump rate and detecting AIL using the initial AIL detection sensitivity level.

While the infusion program runs with the reduced pump rate and the initial AIL detection sensitivity level, the pump determines (616) if a second VTBI threshold TH2 after the initial VTBI threshold is reached (e.g., by sensing an amount of fluid that has been pumped or by determining how full or empty the fluid supply is). The second VTBI threshold TH2 corresponds to an amount of the VTBI pumped (e.g., 98%) higher than the second amount of the VTBI pumped, or an amount of the VTBI remaining to be pumped (e.g., 2%) lower than the second amount of the VTBI remaining to be pumped. When the second VTBI threshold TH2 is reached, the pump (e.g., 132) increases (618) the AIL detection sensitivity (e.g., by lowering an AIL safety threshold and/or shortening an AIL detection sample period) and/or escalates the notification, and continues (620) pumping at the reduced pump rate and detecting AIL using the increased AIL detection sensitivity level until process 600 ends.

Process 600 ends at the earlier of the end of the VTBI (e.g., due to the total volume of the fluid having been pumped), receiving an instruction to stop or pause the program at the pump or at the controller (e.g., by the clinician), or an AIL alarm event (e.g., due to detected air surpassing a safety threshold). Throughout process 600, if an AIL alarm event occurs at any step in the process, the infusion program (and, thus, process 600) stops until a clinician can restart the infusion program (e.g., after checking the administration set for air and/or replacing the fluid supply).

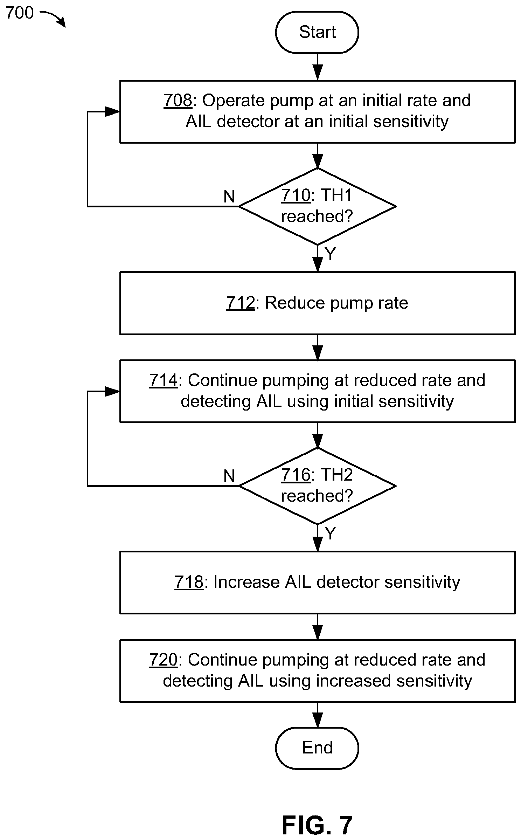

Figure 7 depicts an example AIL prevention process 700 including dynamic adjustment of pump rate and AIL detection sensitivity in accordance with some implementations. Process 700 is similar to process 600, but without notifications or escalations of notifications. As such, only two VTBI thresholds (TH1 and TH2) are measured. Such a process may operate in parallel with a separate program that controls notification, may run according to an infusion program in which notifications are not specified, or may run as a result of the clinician turning off notifications. Operations 708-720 in process 700 correspond to operations 608-620 in process 600 (without the notifications), and are thus not described again here.

In processes 600 and 700 (Figures 6 and 7), nuisance alarm prevention is initially prioritized by keeping the AIL detection sensitivity relatively low, or otherwise at a reasonable rate that optimizes for air detection while minimizing false positives. However, as the infusion program nears the end of the infusion program (after both TH1 and TH2 have reached), nuisance alarm prevention is not as important as protecting patient safety by reducing the increasing risk of air being introduced into the administrative set 122 (e.g., due to the fluid supply 112 running out before the VTBI has been reached). Thus, toward the end of the infusion program (after both TH1 and TH2 have been reached), AIL detection is prioritized by increasing detection sensitivity, even if that means an increase in false positive AIL alarms. These false positive alarms may even be useful in that they could serve as the basis for additional reminders to the clinician when preventative measures should be taken to avoid accidental air in the system (e.g., by switching out the fluid supply 112 before it runs out).

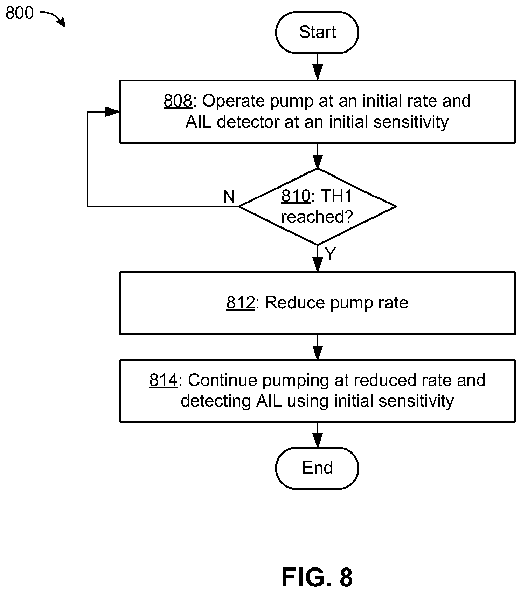

Figure 8 depicts an example AIL prevention process 800 including dynamic adjustment of pump rate in accordance with some implementations. Process 800 is similar to process 700, but without AIL detection sensitivity adjustments. As such, only one VTBI threshold (TH1) is measured. Such a process may operate may run according to an infusion program in which notifications and AIL detection sensitivity adjustments are not specified, or may run as a result of the clinician turning off notifications and AIL detection sensitivity adjustments. Operations 808-814 in process 800 correspond to operations 708-714 in process 700 (without the AIL detection sensitivity adjustments), and are thus not described again here.

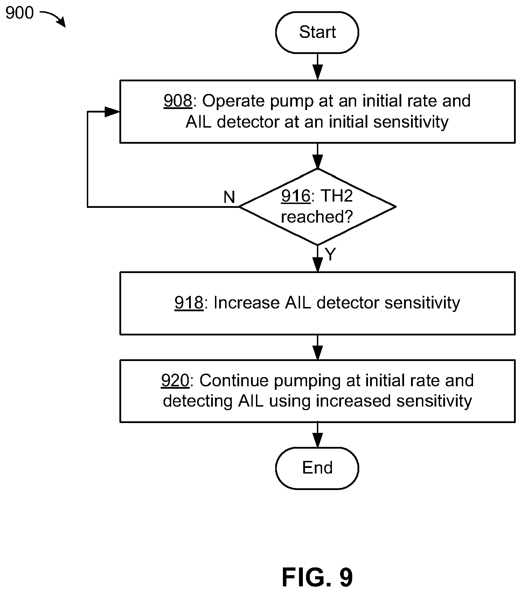

Figure 9 depicts an example AIL prevention process 900 including dynamic adjustment of AIL detection sensitivity in accordance with some implementations. Process 900 is similar to process 700, but without pump rate adjustments. As such, only one VTBI threshold (TH2) is measured. Such a process may operate may run according to an infusion program in which notifications and pump rate adjustments are not specified, or may run as a result of the clinician turning off notifications and pump rate adjustments. Operations 908 and 916-920 in process 900 correspond to operations 708 and 716-720 in process 700 (without the pump rate adjustments), and are thus not described again here. In process 900, nuisance alarm prevent is balanced with patient safety as discussed above with reference to processes 600 and 700 (Figures 6 and 7).

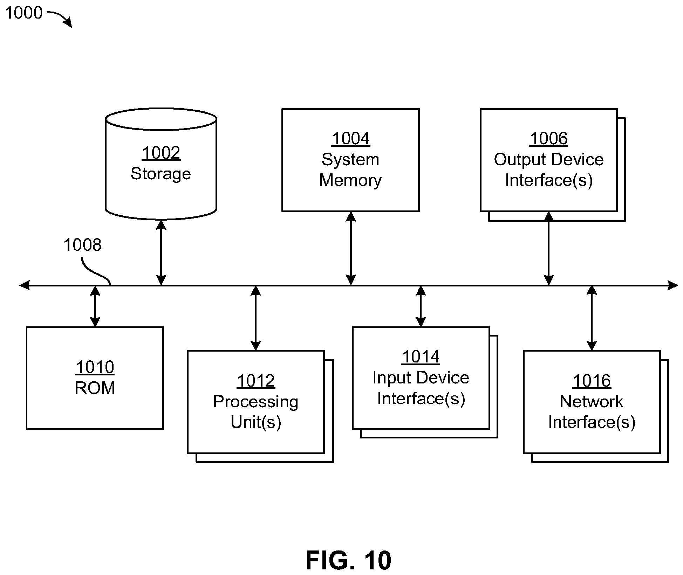

Figure 10 is a conceptual diagram illustrating an example electronic system 1000 in accordance with some implementations. The electronic system 1000 may be implemented by a computing device for execution of software associated with portions or steps of any of the processes 1000-900, or components and methods provided by Figures 1-9. In this regard, the electronic system 1000 may include infusion device 102. The electronic system 1000 may also include a specifically-configured personal computer or a mobile device for infusion such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

The electronic system 1000 may also include various types of computer readable media and interfaces for various other types of computer readable media. In the depicted example, the electronic system 1000 includes a bus 1008, processing unit(s) 1012, a system memory 1004, a read-only memory (ROM) 1010, a permanent storage device 1002, input device interface(s) 1014, output device interface(s) 1006, and network interface(s) 1016. In some implementations, the electronic system 1000 may include or be integrated with other computing devices or circuitry for operation of the various components and methods previously described.

The bus 1008 collectively represents all system, peripheral, and chipset buses that communicatively connect the numerous internal devices of the electronic system 1000. For instance, the bus 1008 communicatively connects the processing unit(s) 1012 with the ROM 1010, the system memory 1004, and the permanent storage device 1002.

From these various memory units, processing unit(s) 1012 retrieves instructions to execute and data to process in order to execute the processes of the subject disclosure. The processing unit(s) 1012 can be a single processor or a multi-core processor in different implementations.

The ROM 1010 stores static data and instructions that are needed by the processing unit(s) 1012 and other modules of the electronic system. The permanent storage device 1002, on the other hand, is a read-and-write memory device. This device is a non-volatile memory unit that stores instructions and data even when the electronic system 1000 is powered off. Some implementations of the subject disclosure use a mass-storage device (such as a magnetic or optical disk and its corresponding disk drive) as the permanent storage device 1002. Other implementations use a removable storage device (such as a floppy disk, flash drive, and its corresponding disk drive) as the permanent storage device 1002.

Like the permanent storage device 1002, the system memory 1004 is a read-and-write memory device. However, unlike the storage device 1002, the system memory 1004 is a volatile read-and-write memory, such as random-access memory (RAM). The system memory 1004 stores some of the instructions and data that the processor needs at runtime. In some implementations, the processes of the subject disclosure are stored in the system memory 1004, the permanent storage device 1002, and/or the ROM 1010. From these various memory units, the processing unit(s) 1012 retrieves instructions to execute and data to process, in order to execute the processes of some implementations.

The bus 1008 also connects to the input device interface(s) 1014 and the output device interface(s) 1006. The input device interface(s) 1014 enables the user to communicate information and select commands to the electronic system. Input devices used with the input device interface(s) 1014 include, for example, alphanumeric keyboards and pointing devices (also called "cursor control devices"). The output device interface(s) 1006 enables, for example, the display of images generated by the electronic system 1000. Output devices used with the output device interface(s) 1006 include, for example, printers and display devices, such as cathode ray tubes (CRT) or liquid crystal displays (LCD). Some implementations include devices (e.g., touchscreens) that function as both input and output devices.

Furthermore, the bus 1008 also couples the electronic system 1000 to a network (not shown) through the network interface(s) 1016. The network interface(s) 1016 may include, for example, a wireless access point (e.g., Bluetooth or Wi-Fi) or radio circuitry for connecting to a wireless access point. The network interface(s) 1016 may also include hardware (e.g., ethernet hardware) for connecting the computer to a part of a network of computers such as a local area network (LAN), a wide area network (WAN), wireless LAN, an intranet, or a network of networks, such as the Internet. Any or all components of electronic system 1000 can be used in conjunction with the subject disclosure when specifically configured with one of more of the features described.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field-programmable gate arrays (FPGAs). In some implementations, such integrated circuits execute instructions that are stored on the circuit itself.

As used in this specification and any claims of this application, the terms "computer," "server," "processor," and "memory" all refer to electronic or other technological devices specifically configured with one or more of the features described above. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well. For example, feedback provided to the user can be any form of sensory feedback (e.g., visual feedback, auditory feedback, tactile feedback), and input from the user can be received in forms such as acoustic, speech, gesture, or tactile input. In addition, a computer can interact with a user by sending documents to and receiving documents from a device that is used by the user (e.g., by sending web pages to a web browser on a user's client device in response to requests received from the web browser).

Implementations of the subject matter described in this specification can be implemented in a specifically configured computing system that includes a back end component (e.g., a data server), or that includes a specifically configured middleware component (e.g., an application server), or that includes a specifically configured front end component (e.g., a client computer having a graphical user interface or a Web browser through which a user can interact with an implementation of the subject matter described in this specification), or any combination of one or more such back end, middleware, or front end components. The components of the system can be interconnected by one or more forms or mediums of digital data communication, such as a communication network. Examples of communication networks include a LAN and a WAN, an inter-network (e.g., the Internet), and peer-to-peer networks (e.g., ad hoc peer-to-peer networks).

The computing system can include specifically configured clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art will appreciate that the various illustrative blocks, modules, elements, components, methods, and algorithms described herein may be implemented as electronic hardware, computer software, or a combination thereof. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order and are not meant to be limited to the specific order or hierarchy presented.

### Further Consideration:

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects.

Thus, the claims are not intended to be limited to the aspects shown herein but are to be accorded the full scope consistent with the language of the claims. For example, reference to an element in the singular is not intended to mean "one and only one" unless specifically so stated, but rather "one or more." Moreover, unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to," "operable to," and "programmed to" do not imply any particular tangible or intangible modification of a subject, but, rather, are intended to be used interchangeably. For example, a processor configured to monitor and control an operation or a component, may also mean the processor being programmed to monitor and control the operation or the processor being operable to monitor and control the operation. Likewise, a processor configured to execute code can be construed as a processor programmed to execute code or operable to execute code.

The term "automatic," as used herein, may include performance by a computer or machine without user intervention, for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An implementation may provide one or more examples. A phrase such as an "implementation" may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface, or a UI) may refer to a network-based interface including data fields or other control elements for receiving input signals or providing electronic information or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database, or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine-readable aggregation of information such as an XML document, fixed field message, comma separated message, JSON, a custom mode, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As used herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In some implementations, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. An infusion device (102), comprising:
a pumping element (202) configured to pump a fluid through a fluid tube into a patient connector;
an air-in-line sensor (210) configured to measure air within the fluid tube;
a display;
a processor; and
a non-transitory computer readable medium comprising instructions that, when executed by the processor, cause the infusion device to:
receive an instruction indicating a total volume to be infused, VTBI, of the fluid;
cause the pumping element to pump the fluid through the fluid tube according to a pumping rate and subject to a safety threshold amount of air within the fluid tube; and
cause the air-in-line sensor to measure an amount of the air within the fluid tube according to a sensitivity level;
determine that a first VTBI threshold has been met, the first VTBI threshold corresponding to a first predetermined amount of the VTBI having been pumped or remaining to be pumped;
in response to determining that the first VTBI threshold has been met, output a notification on the display or reduce the pumping rate of the pumping element;
after outputting the notification or reducing the pumping rate, determine that a second VTBI threshold has been met, the second VTBI threshold corresponding to a second predetermined amount of the VTBI having been pumped or remaining to be pumped; and
in response to determining that the first and second VTBI thresholds have been met, increase the sensitivity level of the air-in-line sensor.

2. The infusion device of claim 1, wherein the instructions further cause the infusion device to:
after increasing the sensitivity level of the air-in-line sensor, cause the pumping element to continue pumping the fluid through the fluid tube until the air-in-line sensor, using the increased sensitivity level, measures an amount of air within the fluid tube that exceeds the safety threshold.

3. The infusion device of claim 1 or claim 2, wherein:
the safety threshold corresponds to a range of air measurements or an absolute air measurement; and
the safety threshold is based on a drug or medication type of the fluid or an ambient condition proximate to the infusion device.

4. The infusion device of any one of claims 1-3, wherein the instructions further cause the infusion device to:
determine that the amount of air in the fluid tube exceeds the safety threshold; and
in response to determining that the amount of air in the fluid tube exceeds the safety threshold, output an escalated notification on the display or pause pumping of the fluid through the fluid tube.

5. The infusion device of any one of claims 1-4, wherein the instructions further cause the infusion device to:
determine that the amount of air in the fluid does not exceed the safety threshold; and
in response to determining that the amount of air in the fluid tube does not exceed the safety threshold, cause the pumping element to continue pumping the fluid through the fluid tube according to the instruction indicating the total VTBI.

6. The infusion device of any one of claims 1-5, wherein the instructions that cause the infusion device to increase the sensitivity level of the air-in-line sensor include instructions that cause the infusion device to lower the safety threshold.

7. The infusion device of any one of claims 1-6, wherein the instructions that cause the infusion device to increase the sensitivity level of the air-in-line sensor include instructions that cause the infusion device to shorten a sampling size used by the air-in-line sensor.

8. The infusion device of any one of claims 1-7, wherein the instructions that cause the air-in-line sensor to measure the amount of air in the fluid tube include instructions that cause the air-in-line sensor to measure a rolling average amount of air in the fluid tube, an accumulated amount of air in the fluid tube, a number of air bubbles in the fluid tube, or an estimated volume of air in the fluid tube.

9. The infusion device of any one of claims 1-8, wherein:
the first VTBI threshold corresponds to between 90% and 97% of the VTBI having been pumped or between 3% and 10% of the VTBI remaining to be pumped; and
the second VTBI threshold corresponds to between 95% and 99% of the VTBI having been pumped or between 1% and 5% of the VTBI remaining to be pumped.

10. The infusion device of any one of claims 1-9, wherein the first VTBI threshold and the second VTBI threshold are configurable based on a drug or medication type of the fluid or an infusion type associated with the pumping of the fluid.

11. A method of operating an infusion device to reduce air-in-line alarms, the method comprising:
receiving an instruction at a processor of the infusion device indicating a total volume to be infused, VTBI, of a fluid for a pumping element of the infusion device to pump through a fluid tube into a patient connector;
causing the pumping element to pump the fluid through the fluid tube according to a pumping rate and subject to a safety threshold amount of air within the fluid tube;
causing an air-in-line sensor of the infusion device to measure an amount of air within the fluid tube according to a sensitivity level;
determining that a first VTBI threshold has been met, the first VTBI threshold corresponding to a first predetermined amount of the VTBI having been pumped or remaining to be pumped;
in response to determining that the first VTBI threshold has been met, outputting a notification on the display or reducing the pumping rate of the pumping element;
after outputting the notification or reducing the pumping rate, determining that a second VTBI threshold has been met, the second VTBI threshold corresponding to a second predetermined amount of the VTBI having been pumped or remaining to be pumped; and
in response to determining that the first and second VTBI thresholds have been met, increasing the sensitivity level of the air-in-line sensor.

12. The method of claim 11, further comprising:
after increasing the sensitivity level of the air-in-line sensor, causing the pumping element to continue pumping the fluid through the fluid tube until the air-in-line sensor, using the increased sensitivity level, measures an amount of air within the fluid tube that exceeds the safety threshold.

13. The method of claim 11 or claim 12, further comprising:
obtaining a drug or medication type of the fluid or an ambient condition proximate to the infusion device; and
setting the safety threshold based on the drug or medication type of the fluid or the ambient condition proximate to the infusion device.

14. The method of any one of claims 11-13, further comprising:
determining that the amount of air in the fluid tube exceeds the safety threshold; and
in response to determining that the amount of air in the fluid tube exceeds the safety threshold, outputting an escalated notification on the display or pause pumping of the fluid through the fluid tube.

15. The method of any one of claims 11-14, further comprising:
determining that the amount of air in the fluid does not exceed the safety threshold; and
in response to determining that the amount of air in the fluid tube does not exceed the safety threshold, causing the pumping element to continue pumping the fluid through the fluid tube according to the instruction indicating the total VTBI.

16. The method of any one of claims 11-15, wherein increasing the sensitivity level of the air-in-line sensor includes lowering the safety threshold.

17. The method of any one of claims 11-16, wherein increasing the sensitivity level of the air-in-line sensor includes shortening a sampling size used by the air-in-line sensor.

18. The method of any one of claims 11-17, wherein measuring the amount of air in the fluid tube includes measuring a rolling average amount of air in the fluid tube, an accumulated amount of air in the fluid tube, a number of air bubbles in the fluid tube, or an estimated volume of air in the fluid tube.

19. The method of any one of claims 11-18, wherein:
the first VTBI threshold corresponds to between 90% and 97% of the VTBI having been pumped or between 3% and 10% of the VTBI remaining to be pumped; and
the second VTBI threshold corresponds to between 95% and 99% of the VTBI having been pumped or between 1% and 5% of the VTBI remaining to be pumped.

20. The method of any one of claims 11-19, further comprising setting at least one of the first VTBI threshold and the second VTBI threshold based on a drug or medication type of the fluid or an infusion type associated with the pumping of the fluid.

## Patentansprüche

1. Infusionsvorrichtung (102), umfassend:
ein Pumpenelement (202), das dazu konfiguriert ist, ein Fluid durch einen Fluidschlauch in einen Patientenkonnektor zu pumpen;
einen Luft-in-Leitung-Sensor (210), der dazu konfiguriert ist, Luft innerhalb des Fluidschlauchs zu messen;
eine Anzeigeeinrichtung;
einen Prozessor; und
ein nichtflüchtiges computerlesbares Medium, das Anweisungen umfasst, die, wenn sie durch den Prozessor ausgeführt werden, bewirken, dass die Infusionsvorrichtung:
eine Anweisung empfängt, die ein insgesamt zu infundierendes Volumen, VTBI, des Fluids angibt;
bewirkt, dass das Pumpenelement das Fluid durch den Fluidschlauch gemäß einer Pumprate und vorbehaltlich einer Sicherheitsschwelle für eine Luftmenge innerhalb des Fluidschlauchs pumpt; und
bewirkt, dass der Luft-in-Leitung-Sensor eine Luftmenge innerhalb des Fluidschlauchs gemäß einem Empfindlichkeitsniveau misst;
feststellt, dass eine erste VTBI-Schwelle erreicht worden ist, wobei die erste VTBI-Schwelle einer ersten vorbestimmten Menge des VTBI entspricht, die gepumpt worden ist oder noch zu pumpen ist;
als Reaktion auf das Feststellen, dass die erste VTBI-Schwelle erreicht worden ist, eine Benachrichtigung auf der Anzeigeeinrichtung ausgibt oder die Pumprate des Pumpenelements reduziert;
nach dem Ausgeben der Benachrichtigung oder dem Reduzieren der Pumprate feststellt, dass eine zweite VTBI-Schwelle erreicht worden ist, wobei die zweite VTBI-Schwelle einer zweiten vorbestimmten Menge des VTBI entspricht, die gepumpt worden ist oder noch zu pumpen ist; und
als Reaktion auf das Feststellen, dass die erste und die zweite VTBI-Schwelle erreicht worden sind, das Empfindlichkeitsniveau des Luft-in-Leitung-Sensors erhöht.

2. Infusionsvorrichtung nach Anspruch 1, wobei die Anweisungen ferner bewirken, dass die Infusionsvorrichtung:
nach dem Erhöhen des Empfindlichkeitsniveaus des Luft-in-Leitung-Sensors bewirkt, dass das Pumpenelement das Fluid durch den Fluidschlauch weiter pumpt, bis der Luft-in-Leitung-Sensor unter Verwendung des erhöhten Empfindlichkeitsniveaus eine Luftmenge innerhalb des Fluidschlauchs misst, die die Sicherheitsschwelle überschreitet.

3. Infusionsvorrichtung nach Anspruch 1 oder Anspruch 2, wobei:
die Sicherheitsschwelle einem Bereich von Luftmessungen oder einer absoluten Luftmessung entspricht; und
die Sicherheitsschwelle auf einem Arzneimittel- oder Medikamententyp des Fluids oder einer Umgebungsbedingung in der Nähe der Infusionsvorrichtung basiert.

4. Infusionsvorrichtung nach einem der Ansprüche 1-3, wobei die Anweisungen ferner bewirken, dass die Infusionsvorrichtung:
feststellt, dass die Luftmenge im Fluidschlauch die Sicherheitsschwelle überschreitet; und
als Reaktion auf das Feststellen, dass die Luftmenge im Fluidschlauch die Sicherheitsschwelle überschreitet, eine höherstufige Benachrichtigung auf der Anzeigeeinrichtung ausgibt oder das Pumpen des Fluids durch den Fluidschlauch unterbricht.

5. Infusionsvorrichtung nach einem der Ansprüche 1-4, wobei die Anweisungen ferner bewirken, dass die Infusionsvorrichtung:
feststellt, dass die Luftmenge im Fluid die Sicherheitsschwelle nicht überschreitet; und
als Reaktion auf das Feststellen, dass die Luftmenge im Fluidschlauch die Sicherheitsschwelle nicht überschreitet, bewirkt, dass das Pumpenelement das Fluid durch den Fluidschlauch gemäß der Anweisung, die das gesamte VTBI angibt, weiter pumpt.

6. Infusionsvorrichtung nach einem der Ansprüche 1-5, wobei die Anweisungen, die bewirken, dass die Infusionsvorrichtung das Empfindlichkeitsniveau des Luft-in-Leitung-Sensors erhöht, Anweisungen umfassen, die bewirken, dass die Infusionsvorrichtung die Sicherheitsschwelle absenkt.

7. Infusionsvorrichtung nach einem der Ansprüche 1-6, wobei die Anweisungen, die bewirken, dass die Infusionsvorrichtung das Empfindlichkeitsniveau des Luft-in-Leitung-Sensors erhöht, Anweisungen umfassen, die bewirken, dass die Infusionsvorrichtung einen durch den Luft-in-Leitung-Sensor verwendeten Abtastumfang verkürzt.

8. Infusionsvorrichtung nach einem der Ansprüche 1-7, wobei die Anweisungen, die bewirken, dass der Luft-in-Leitung-Sensor die Luftmenge im Fluidschlauch misst, Anweisungen umfassen, die bewirken, dass der Luft-in-Leitung-Sensor eine gleitend gemittelte Luftmenge im Fluidschlauch, eine akkumulierte Luftmenge im Fluidschlauch, eine Anzahl von Luftblasen im Fluidschlauch oder ein geschätztes Luftvolumen im Fluidschlauch misst.

9. Infusionsvorrichtung nach einem der Ansprüche 1-8, wobei:
die erste VTBI-Schwelle einem Anteil zwischen 90 % und 97 % des VTBI entspricht, der gepumpt worden ist, oder einem Anteil zwischen 3 % und 10 % des VTBI entspricht, der noch zu pumpen ist; und
die zweite VTBI-Schwelle einem Anteil zwischen 95 % und 99 % des VTBI entspricht, der gepumpt worden ist, oder einem Anteil zwischen 1 % und 5 % des VTBI entspricht, der noch zu pumpen ist.

10. Infusionsvorrichtung nach einem der Ansprüche 1-9, wobei die erste VTBI-Schwelle und die zweite VTBI-Schwelle auf der Grundlage eines Arzneimittel- oder Medikamententyps des Fluids oder eines mit dem Pumpen des Fluids verbundenen Infusionstyps konfigurierbar sind.

11. Verfahren zum Betreiben einer Infusionsvorrichtung zur Reduzierung von Luft-in-Leitung-Alarmen, wobei das Verfahren umfasst:
Empfangen einer Anweisung an einem Prozessor der Infusionsvorrichtung, die ein insgesamt zu infundierendes Volumen, VTBI, eines Fluids angibt, das durch ein Pumpenelement der Infusionsvorrichtung durch einen Fluidschlauch in einen Patientenkonnektor zu pumpen ist;
Bewirken, dass das Pumpenelement das Fluid durch den Fluidschlauch gemäß einer Pumprate und vorbehaltlich einer Sicherheitsschwelle für eine Luftmenge innerhalb des Fluidschlauchs pumpt;
Bewirken, dass ein Luft-in-Leitung-Sensor der Infusionsvorrichtung eine Luftmenge innerhalb des Fluidschlauchs gemäß einem Empfindlichkeitsniveau misst;
Feststellen, dass eine erste VTBI-Schwelle erreicht worden ist, wobei die erste VTBI-Schwelle einer ersten vorbestimmten Menge des VTBI entspricht, die gepumpt worden ist oder noch zu pumpen ist;
als Reaktion auf das Feststellen, dass die erste VTBI-Schwelle erreicht worden ist, Ausgeben einer Benachrichtigung auf der Anzeigeeinrichtung oder Reduzieren der Pumprate des Pumpenelements;
nach dem Ausgeben der Benachrichtigung oder dem Reduzieren der Pumprate Feststellen, dass eine zweite VTBI-Schwelle erreicht worden ist, wobei die zweite VTBI-Schwelle einer zweiten vorbestimmten Menge des VTBI entspricht, die gepumpt worden ist oder noch zu pumpen ist; und
als Reaktion auf das Feststellen, dass die erste und die zweite VTBI-Schwelle erreicht worden sind, Erhöhen des Empfindlichkeitsniveaus des Luft-in-Leitung-Sensors.

12. Verfahren nach Anspruch 11, ferner umfassend:
nach dem Erhöhen des Empfindlichkeitsniveaus des Luft-in-Leitung-Sensors Bewirken, dass das Pumpenelement das Fluid durch den Fluidschlauch weiter pumpt, bis der Luft-in-Leitung-Sensor unter Verwendung des erhöhten Empfindlichkeitsniveaus eine Luftmenge innerhalb des Fluidschlauchs misst, die die Sicherheitsschwelle überschreitet.

13. Verfahren nach Anspruch 11 oder Anspruch 12, ferner umfassend:
Ermitteln eines Arzneimittel- oder Medikamententyps des Fluids oder einer Umgebungsbedingung in der Nähe der Infusionsvorrichtung; und
Setzen der Sicherheitsschwelle auf der Grundlage des Arzneimittel- oder Medikamententyps des Fluids oder der Umgebungsbedingung in der Nähe der Infusionsvorrichtung.

14. Verfahren nach einem der Ansprüche 11-13, ferner umfassend:
Feststellen, dass die Luftmenge im Fluidschlauch die Sicherheitsschwelle überschreitet; und
als Reaktion auf das Feststellen, dass die Luftmenge im Fluidschlauch die Sicherheitsschwelle überschreitet, Ausgeben einer höherstufigen Benachrichtigung auf der Anzeigeeinrichtung oder Unterbrechen des Pumpens des Fluids durch den Fluidschlauch.

15. Verfahren nach einem der Ansprüche 11-14, ferner umfassend:
Feststellen, dass die Luftmenge im Fluid die Sicherheitsschwelle nicht überschreitet; und
als Reaktion auf das Feststellen, dass die Luftmenge im Fluidschlauch die Sicherheitsschwelle nicht überschreitet, Bewirken, dass das Pumpenelement das Fluid durch den Fluidschlauch gemäß der Anweisung, die das gesamte VTBI angibt, weiter pumpt.

16. Verfahren nach einem der Ansprüche 11-15, wobei das Erhöhen des Empfindlichkeitsniveaus des Luft-in-Leitung-Sensors das Absenken der Sicherheitsschwelle umfasst.

17. Verfahren nach einem der Ansprüche 11-16, wobei das Erhöhen des Empfindlichkeitsniveaus des Luft-in-Leitung-Sensors das Verkürzen eines durch den Luft-in-Leitung-Sensor verwendeten Abtastumfangs umfasst.

18. Verfahren nach einem der Ansprüche 11-17, wobei das Messen der Luftmenge im Fluidschlauch das Messen einer gleitend gemittelten Luftmenge im Fluidschlauch, einer akkumulierten Luftmenge im Fluidschlauch, einer Anzahl von Luftblasen im Fluidschlauch oder eines geschätzten Luftvolumens im Fluidschlauch umfasst.

19. Verfahren nach einem der Ansprüche 11-18, wobei:
die erste VTBI-Schwelle einem Anteil zwischen 90 % und 97 % des VTBI entspricht, der gepumpt worden ist, oder einem Anteil zwischen 3 % und 10 % des VTBI entspricht, der noch zu pumpen ist; und
die zweite VTBI-Schwelle einem Anteil zwischen 95 % und 99 % des VTBI entspricht, der gepumpt worden ist, oder einem Anteil zwischen 1 % und 5 % des VTBI entspricht, der noch zu pumpen ist.

20. Verfahren nach einem der Ansprüche 11-19, ferner umfassend das Setzen von zumindest einer der ersten VTBI-Schwelle und der zweiten VTBI-Schwelle auf der Grundlage eines Arzneimittel- oder Medikamententyps des Fluids oder eines mit dem Pumpen des Fluids verbundenen Infusionstyps.

## Revendications

1. Dispositif de perfusion (102), comprenant :
un élément de pompage (202) configuré pour pomper un fluide à travers un tube de fluide dans un connecteur patient ;
un capteur d'air en ligne (210) configuré pour mesurer de l'air à l'intérieur du tube de fluide ;
un dispositif d'affichage ;
un processeur ; et
un support lisible par ordinateur non transitoire comprenant des instructions qui, lorsqu'elles sont exécutées par le processeur, amènent le dispositif de perfusion à :
recevoir une instruction indiquant un volume total à perfuser, VTBI, du fluide ;
amener l'élément de pompage à pomper le fluide à travers le tube de fluide conformément à un débit de pompage et sous réserve d'un seuil de sécurité relatif à une quantité d'air à l'intérieur du tube de fluide ; et
amener le capteur d'air en ligne à mesurer une quantité d'air à l'intérieur du tube de fluide conformément à un niveau de sensibilité ;
déterminer qu'un premier seuil VTBI a été atteint, le premier seuil VTBI correspondant à une première quantité prédéterminée du VTBI ayant été pompée ou restant à pomper ;
en réponse à la détermination selon laquelle le premier seuil VTBI a été atteint, émettre une notification sur le dispositif d'affichage ou réduire le débit de pompage de l'élément de pompage ;
après l'émission de la notification ou la réduction du débit de pompage, déterminer qu'un second seuil VTBI a été atteint, le second seuil VTBI correspondant à une seconde quantité prédéterminée du VTBI ayant été pompée ou restant à pomper ; et
en réponse à la détermination selon laquelle les premier et second seuils VTBI ont été atteints, augmenter le niveau de sensibilité du capteur d'air en ligne.

2. Dispositif de perfusion selon la revendication 1, dans lequel les instructions amènent en outre le dispositif de perfusion à :
après l'augmentation du niveau de sensibilité du capteur d'air en ligne, amener l'élément de pompage à continuer à pomper le fluide à travers le tube de fluide jusqu'à ce que le capteur d'air en ligne, en utilisant le niveau de sensibilité augmenté, mesure une quantité d'air à l'intérieur du tube de fluide qui dépasse le seuil de sécurité.

3. Dispositif de perfusion selon la revendication 1 ou la revendication 2, dans lequel :
le seuil de sécurité correspond à une plage de mesures d'air ou à une mesure d'air absolue ; et
le seuil de sécurité est basé sur un type de médicament ou de substance médicamenteuse du fluide ou sur une condition ambiante à proximité du dispositif de perfusion.

4. Dispositif de perfusion selon l'une quelconque des revendications 1 à 3, dans lequel les instructions amènent en outre le dispositif de perfusion à :
déterminer que la quantité d'air dans le tube de fluide dépasse le seuil de sécurité ; et
en réponse à la détermination selon laquelle la quantité d'air dans le tube de fluide dépasse le seuil de sécurité, émettre une notification de niveau supérieur sur le dispositif d'affichage ou suspendre le pompage du fluide à travers le tube de fluide.

5. Dispositif de perfusion selon l'une quelconque des revendications 1 à 4, dans lequel les instructions amènent en outre le dispositif de perfusion à :
déterminer que la quantité d'air dans le fluide ne dépasse pas le seuil de sécurité ; et
en réponse à la détermination selon laquelle la quantité d'air dans le tube de fluide ne dépasse pas le seuil de sécurité, amener l'élément de pompage à continuer à pomper le fluide à travers le tube de fluide conformément à l'instruction indiquant le VTBI total.

6. Dispositif de perfusion selon l'une quelconque des revendications 1 à 5, dans lequel les instructions qui amènent le dispositif de perfusion à augmenter le niveau de sensibilité du capteur d'air en ligne comprennent des instructions qui amènent le dispositif de perfusion à abaisser le seuil de sécurité.

7. Dispositif de perfusion selon l'une quelconque des revendications 1 à 6, dans lequel les instructions qui amènent le dispositif de perfusion à augmenter le niveau de sensibilité du capteur d'air en ligne comprennent des instructions qui amènent le dispositif de perfusion à raccourcir une taille d'échantillonnage utilisée par le capteur d'air en ligne.

8. Dispositif de perfusion selon l'une quelconque des revendications 1 à 7, dans lequel les instructions qui amènent le capteur d'air en ligne à mesurer la quantité d'air dans le tube de fluide comprennent des instructions qui amènent le capteur d'air en ligne à mesurer une quantité moyenne glissante d'air dans le tube de fluide, une quantité accumulée d'air dans le tube de fluide, un nombre de bulles d'air dans le tube de fluide, ou un volume estimé d'air dans le tube de fluide.

9. Dispositif de perfusion selon l'une quelconque des revendications 1 à 8, dans lequel :
le premier seuil VTBI correspond à une proportion comprise entre 90 % et 97 % du VTBI ayant été pompée ou à une proportion comprise entre 3 % et 10 % du VTBI restant à pomper ; et
le second seuil VTBI correspond à une proportion comprise entre 95 % et 99 % du VTBI ayant été pompée ou à une proportion comprise entre 1 % et 5 % du VTBI restant à pomper.

10. Dispositif de perfusion selon l'une quelconque des revendications 1 à 9, dans lequel le premier seuil VTBI et le second seuil VTBI sont configurables sur la base d'un type de médicament ou de substance médicamenteuse du fluide ou d'un type de perfusion associé au pompage du fluide.

11. Procédé d'exploitation d'un dispositif de perfusion afin de réduire des alarmes d'air en ligne, le procédé comprenant :
recevoir une instruction au niveau d'un processeur du dispositif de perfusion indiquant un volume total à perfuser, VTBI, d'un fluide à pomper, par un élément de pompage du dispositif de perfusion, à travers un tube de fluide dans un connecteur patient ;
amener l'élément de pompage à pomper le fluide à travers le tube de fluide conformément à un débit de pompage et sous réserve d'un seuil de sécurité relatif à une quantité d'air à l'intérieur du tube de fluide ;
amener un capteur d'air en ligne du dispositif de perfusion à mesurer une quantité d'air à l'intérieur du tube de fluide conformément à un niveau de sensibilité ;
déterminer qu'un premier seuil VTBI a été atteint, le premier seuil VTBI correspondant à une première quantité prédéterminée du VTBI ayant été pompée ou restant à pomper ;
en réponse à la détermination selon laquelle le premier seuil VTBI a été atteint, émettre une notification sur le dispositif d'affichage ou réduire le débit de pompage de l'élément de pompage ;
après l'émission de la notification ou la réduction du débit de pompage, déterminer qu'un second seuil VTBI a été atteint, le second seuil VTBI correspondant à une seconde quantité prédéterminée du VTBI ayant été pompée ou restant à pomper ; et
en réponse à la détermination selon laquelle les premier et second seuils VTBI ont été atteints, augmenter le niveau de sensibilité du capteur d'air en ligne.

12. Procédé selon la revendication 11, comprenant en outre :
après l'augmentation du niveau de sensibilité du capteur d'air en ligne, amener l'élément de pompage à continuer à pomper le fluide à travers le tube de fluide jusqu'à ce que le capteur d'air en ligne, en utilisant le niveau de sensibilité augmenté, mesure une quantité d'air à l'intérieur du tube de fluide qui dépasse le seuil de sécurité.

13. Procédé selon la revendication 11 ou la revendication 12, comprenant en outre :
obtenir un type de médicament ou de substance médicamenteuse du fluide ou une condition ambiante à proximité du dispositif de perfusion ; et
régler le seuil de sécurité sur la base du type de médicament ou de substance médicamenteuse du fluide ou de la condition ambiante à proximité du dispositif de perfusion.

14. Procédé selon l'une quelconque des revendications 11 à 13, comprenant en outre :
déterminer que la quantité d'air dans le tube de fluide dépasse le seuil de sécurité ; et
en réponse à la détermination selon laquelle la quantité d'air dans le tube de fluide dépasse le seuil de sécurité, émettre une notification de niveau supérieur sur le dispositif d'affichage ou suspendre le pompage du fluide à travers le tube de fluide.

15. Procédé selon l'une quelconque des revendications 11 à 14, comprenant en outre :
déterminer que la quantité d'air dans le fluide ne dépasse pas le seuil de sécurité ; et
en réponse à la détermination selon laquelle la quantité d'air dans le tube de fluide ne dépasse pas le seuil de sécurité, amener l'élément de pompage à continuer à pomper le fluide à travers le tube de fluide conformément à l'instruction indiquant le VTBI total.

16. Procédé selon l'une quelconque des revendications 11 à 15, dans lequel l'augmentation du niveau de sensibilité du capteur d'air en ligne comprend l'abaissement du seuil de sécurité.

17. Procédé selon l'une quelconque des revendications 11 à 16, dans lequel l'augmentation du niveau de sensibilité du capteur d'air en ligne comprend le raccourcissement d'une taille d'échantillonnage utilisée par le capteur d'air en ligne.

18. Procédé selon l'une quelconque des revendications 11 à 17, dans lequel la mesure de la quantité d'air dans le tube de fluide comprend la mesure d'une quantité moyenne glissante d'air dans le tube de fluide, d'une quantité accumulée d'air dans le tube de fluide, d'un nombre de bulles d'air dans le tube de fluide, ou d'un volume estimé d'air dans le tube de fluide.

19. Procédé selon l'une quelconque des revendications 11 à 18, dans lequel :
le premier seuil VTBI correspond à une proportion comprise entre 90 % et 97 % du VTBI ayant été pompée ou à une proportion comprise entre 3 % et 10 % du VTBI restant à pomper ; et
le second seuil VTBI correspond à une proportion comprise entre 95 % et 99 % du VTBI ayant été pompée ou à une proportion comprise entre 1 % et 5 % du VTBI restant à pomper.

20. Procédé selon l'une quelconque des revendications 11 à 19, comprenant en outre le réglage d'au moins l'un des premier et second seuils VTBI sur la base d'un type de médicament ou de substance médicamenteuse du fluide ou d'un type de perfusion associé au pompage du fluide.
